(19) 

Europäisches
Patentamt

European
Patent Office

Office européen
des brevets

(11) **EP 4 667 561 A1**

(12) **EUROPEAN PATENT APPLICATION**
published in accordance with Art. 153(4) EPC

(43) Date of publication:
**24.12.2025  Bulletin 2025/52**

(21) Application number: **24757332.2**

(22) Date of filing: **16.02.2024**

(51) International Patent Classification (IPC):
*C12N 1/20* (2006.01)    *A61K 35/744* (2015.01)
*A61P 37/08* (2006.01)    *A61P 3/10* (2006.01)
*A23L 33/135* (2016.01)    *A61K 8/99* (2017.01)
*A61Q 19/00* (2006.01)    *C12R 1/46* (2006.01)

(52) Cooperative Patent Classification (CPC):
**A23L 33/135; A61K 8/99; A61K 35/744;**
**A61P 3/10; A61P 37/08; A61Q 19/00; C12N 1/20;**
C12R 2001/46

(86) International application number:
**PCT/KR2024/095338**

(87) International publication number:
**WO 2024/172621 (22.08.2024 Gazette 2024/34)**

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**GE KH MA MD TN**

(30) Priority: **17.02.2023  KR 20230021236**
**15.02.2024  KR 20240022080**

(71) Applicant: **Kim, Won Yong**
**Seoul 04378 (KR)**

(72) Inventors:
• **KIM, Kiyoung**
**Seoul 04306 (KR)**
• **KIM, Won Yong**
**Seoul 04378 (KR)**

(74) Representative: **BCKIP Part mbB**
**MK1**
**Landsbergerstraße 98, 3.Stock**
**80339 München (DE)**

Remarks:
The complete document including Reference
Table(s) and the Sequence Listing(s) can be
downloaded from the EPO website

(54) **NOVEL LACTOCOCCUS LACTIS SUBSP. LACTIS STRAIN AND USE THEREOF**

(57)    The present invention relates to a novel strain of *Lactococcus lactis subsp. lactis* LB1022 strain and uses thereof. The *Lactococcus lactis subsp. lactis* LB1022 strain, which is a novel strain isolated from fermented dairy products, is a useful functional lactic acid bacterium with excellent acid tolerance, bile tolerance, and intestinal epithelial cell adhesion ability, and inhibits the production of any one inflammatory factor selected from the group consisting of nitric oxide (NO), tumor necrosis factor-$\alpha$ (TNF-$\alpha$), interferon-$\gamma$ (IFN-$\gamma$), and interleukin-1$\beta$ (IL-1$\beta$), and inhibits the gene expression of p65 or p50 in the nuclear factor kappa B pathway (NF-kB pathway), thereby exhibiting an excellent anti-inflammatory effect. Therefore, a composition comprising, as active ingredients, live bacteria, dead bacteria, or a culture medium thereof of the *Lactococcus lactis subsp. lactis* LB1022 strain may be utilized for the prevention, treatment, or amelioration of inflammatory diseases.

FIG. 1

EP 4 667 561 A1

EP 4 667 561 A1

## Description

### THECHNICAL FIELD

The present invention relates to a novel strain of *Lactococcus lactis subsp. lactis* LB1022 and uses thereof.

### BACKGROUND OF THE INVENTION

Inflammation is a local defense mechanism caused by external or internal stimuli, such as physical trauma, harmful chemicals, infection by microorganisms, or irritants in the body's metabolites. Inflammation is triggered by a variety of mediators produced by damaged tissues and migrating cells, and the response varies depending on the cause of the inflammation. Normally, the inflammatory response removes or neutralizes the cause of the disease and restores normal function to damaged tissues or cells, but in some cases, the inflammatory response is abnormally excessive.

Inflammatory bowel disease, one of the most common inflammatory diseases, is a condition that causes chronic inflammation and ulceration of the intestinal tract, with flare-ups and relapses. Environmental factors, genetics, and an overactive immune response caused by gut bacteria have been reported to be the main causes of inflammatory bowel disease. Inflammatory bowel diseases include ulcerative colitis and Crohn's disease, which have similar symptoms, prognosis, and treatment processes, and are collectively diagnosed as inflammatory bowel disease. In recent years, inflammatory bowel disease has been increasingly diagnosed in people in their 20s and 40s due to westernized diets and lifestyle changes.

Immunosuppressive drugs are often prescribed to treat inflammatory diseases, but these drugs can cause side effects such as nausea, heartburn, headache, dizziness, anemia, and skin rashes, so there is a need to develop new treatment methods that show therapeutic effects from natural materials without side effects.

Lactic acid bacteria, on the other hand, are gut bacteria that live in symbiosis with the human digestive system, breaking down fiber and complex proteins into important nutritional components. For lactobacilli to be useful in the gut, they must be able to survive in low pH environments, such as stomach acid, and be resistant to bile, such as in the small intestine. Depending on the environment of the gut microbiota, the gastrointestinal tract of the host can be improved, and recently, functional lactic acid bacteria have been reported to exhibit various bioactivities, such as improving the symptoms of gastrointestinal diseases

### DETAILED DESCRIPTION OF THE INVENTION

#### TECHNICAL PROBLEM

The present invention relates to a novel strain of *Lactococcus lactis subsp. lactis* LB1022 and uses thereof, and the novel *strain Lactococcus lactis subsp. lactis* LB1022, which was isolated from fermented dairy products, is a useful functional lactic acid bacterium with excellent acid tolerance, bile tolerance, and intestinal epithelial cell adhesion ability, and inhibits the production of any one inflammatory factor selected from the group consisting of nitric oxide (NO), tumor necrosis factor $\alpha$ (TNF $\alpha$), interferon $\gamma$ (IFN $\gamma$), and interleukin 1$\beta$ (IL 1$\beta$), and, by inhibiting the gene expression of p65 or p50 in the Nuclear Factor Kappa B (NF $\kappa$B) pathway, exhibits excellent anti-inflammatory effects, and therefore a composition comprising live bacteria, dead bacteria, or a culture medium thereof of *the Lactococcus lactis subsp. lactis* LB1022 strain as an active ingredient can be utilized for the prevention, treatment, or amelioration of inflammatory diseases.

#### TECHNICAL SOLUTION

The present invention provides *Lactococcus lactis subsp. lactis* strain LB1022, deposited under accession number KCTC 15324BP.

The present invention also provides a pharmaceutical composition for the prevention or treatment of inflammatory diseases, comprising as an active ingredient, live bacteria, dead bacteria, or a culture medium thereof of the strain. The present invention also provides a health functional food composition for the prevention or amelioration of inflammatory diseases, comprising as an active ingredient, live bacteria, dead bacteria, or a culture medium thereof of the strain.

The present invention also provides a health functional food composition for the prevention or amelioration of hangovers, comprising as an active ingredient, live bacteria, dead bacteria, or a culture medium thereof of the strain.

The present invention also provides a health functional food composition the prevention or amelioration of liver function, comprising as an active ingredient, live bacteria, dead bacteria, or a culture medium thereof of the strain.

The present invention also provides a pharmaceutical composition for the prevention or treatment of allergic diseases, comprising as an active ingredient, live bacteria, dead bacteria or a culture medium thereof of the strain. The present invention also provides a health functional food composition for the prevention or amelioration of allergic diseases,

2

comprising as an active ingredient, live bacteria, dead bacteria or a culture medium thereof of the strain.

The present invention also provides a pharmaceutical composition for the prevention or treatment of skin damage, comprising as an active ingredient, live bacteria, dead bacteria, or a culture medium thereof of the strain. The present invention also provides a cosmetic composition for preventing or improving skin damage, comprising as an active ingredient, live bacteria, dead bacteria or a culture medium thereof of the strain.

The present invention also provides a pharmaceutical composition for the prevention or treatment of obesity, comprising as an active ingredient, live bacteria, dead bacteria, or a culture medium thereof of the strain. The present invention also provides a health functional food composition for the prevention or amelioration of obesity, comprising as an active ingredient, live bacteria, dead bacteria or a culture medium thereof of the strain.

The present invention also provides a pharmaceutical composition for the prevention or treatment of diabetes, comprising as an active ingredient, live bacteria, dead bacteria or a culture medium thereof of the strain. The present invention also provides a health functional food composition for the prevention or amelioration of diabetes, comprising as an active ingredient, live bacteria, dead bacteria or a culture medium thereof of the strain.

The present invention also provides a pharmaceutical composition for the prevention or treatment of muscle loss, comprising as an active ingredient, live bacteria, dead bacteria or a culture medium thereof of the strain. The present invention also provides a health functional food composition for the prevention or amelioration of muscle loss, comprising as an active ingredient, live bacteria, dead bacteria, or a culture medium thereof of the strain.

The present invention also provides a health functional food composition for gastrointestinal protection, comprising as an active ingredient, live bacteria, dead bacteria or a culture medium thereof of the strain.

## ADVANTAGEOUS EFFECT

According to the present invention, the novel strain of *Lactococcus lactis subsp. lactis* LB1022, which was isolated from fermented dairy products, is a useful functional lactic acid bacterium with excellent acid tolerance, bile tolerance, and intestinal epithelial cell adhesion ability, and inhibits the production of any one inflammatory factor selected from the group consisting of nitric oxide (NO), tumor necrosis factor $\alpha$ (TNF $\alpha$), interferon $\gamma$ (IFN $\gamma$), and interleukin 1$\beta$ (IL 1$\beta$), and, by inhibiting the gene expression of p65 or p50 in the Nuclear Factor Kappa B(NF $\kappa$B) pathway, exhibits excellent anti-inflammatory effects, and therefore a composition comprising live bacteria, dead bacteria or a culture medium thereof of the *Lactococcus lactis subsp. lactis* LB1022 strain as an active ingredient can be utilized for the prevention, treatment, or amelioration of inflammatory diseases.

## BRIEF DESCRIPTION OF THE DRAWINGS

FIG. 1 is a graph analyzing the bile and acid tolerance of a novel strain of *Lactococcus lactis subsp. lactis* LB1022.

FIG. 2 is a graph analyzing the adhesion ability of the novel strain, *Lactococcus lactis subsp. lactis* LB1022, to intestinal epithelial cells.

FIG. 3a is a graph analyzing the level of Nitric oxide (NO) production in RAW264.7 cells induced with an inflammatory response to evaluate the anti-inflammatory effect of the novel strain, *Lactococcus lactis subsp. lactis* LB1022.

FIG. 3b is a graph analyzing the level of pro-inflammatory cytokine secretion in RAW264.7 cells induced with an inflammatory response to evaluate the anti-inflammatory effect of the novel strain, *Lactococcus lactis subsp. lactis* LB1022.

FIG. 3c is a graph analyzing the gene expression levels of NF-kB subunits, p65 and p50, in RAW264.7 cells induced with an inflammatory response to evaluate the anti-inflammatory effect of the novel strain, *Lactococcus lactis subsp. lactis* LB1022.

FIG. 4 is a graph of alcohol and acetaldehyde degradation efficacy by measuring alcohol dihydrogenase (ADH) and acetaldehyde dihydrogenase (ALDH) activities to evaluate the hangover efficacy of the novel strain, *Lactococcus lactis subsp. lactis* LB1022.

FIG. 5a is a graph of blood aspartate aminotransferase (AST) and alanine aminotransferase (ALT) levels, which are indicators of alcoholic liver damage, measured in alcohol-fed SD rats to evaluate the hepatoprotective efficacy of the novel strain, *Lactococcus lactis subsp. lactis* LB1022.

FIG. 5b is a graph showing the expression level of factors involved in hepatoprotection in alcohol-fed SD rats to evaluate the hepatoprotective efficacy of the novel strain, *Lactococcus lactis subsp. lactis* LB1022.

FIG. 6a is a graph showing the determination of serum IgE and mRNA expression level of factors involved in allergic dermatitis to evaluate the efficacy of the novel strain, *Lactococcus lactis subsp. lactis* LB1022, against allergic diseases.

FIG. 6b is a graph showing the determination of serum IgG1, IgG2a, and cytokine expression level in bronchoalveolar lavage fluid (BALF) to evaluate the efficacy of the novel strain, *Lactococcus lactis subsp. lactis* LB1022, against allergic diseases.

FIG. 7 shows the results of utilizing human skin epithelial cells to evaluate the efficacy of the novel strain, *Lactococcus lactis subsp. lactis* LB1022, against skin damage to assess its skin regeneration efficacy.

FIG. 8 shows the results of utilizing C57BL/6 mice to evaluate the efficacy of a novel strain, *Lactococcus lactis subsp. lactis* LB1022, against obesity to assess its efficacy in reducing body fat.

FIG. 9a shows the results of evaluating the effect of inhibiting glycolytic enzyme activity of a novel strain, *Lactococcus lactis subsp. lactis* LB1022, to evaluate its efficacy against diabetes.

FIG. 9b is the result of analyzing the indicators involved in blood glucose reduction utilizing male C57BL/6 mice to evaluate the efficacy of the novel strain, *Lactococcus lactis subsp. lactis* LB1022 against diabetes.

FIG. 10 shows the results of the analysis of indicators involved in muscle differentiation utilizing muscle cells to evaluate the efficacy of the novel strain, *Lactococcus lactis subsp. lactis* LB1022, against muscle loss.

FIG. 11 shows the results of an analysis of indicators involved in gastrointestinal protection utilizing gastrointestinal epithelial cells to evaluate the efficacy of a novel strain, *Lactococcus lactis subsp. lactis* LB1022, on gastrointestinal protection.

FIG. 12 shows the results of the analysis of indicators involved in gouty inflammation utilizing human-derived macrophages to evaluate the efficacy of the novel strain, *Lactococcus lactis subsp. lactis* LB1022, against gout.

FIG. 13 shows the results of analyzing the effect of the novel strain, *Lactococcus lactis subsp. lactis* LB1022, on reducing corticosterone and reducing the expression level of stress-related factors in adipose tissue utilizing male C57BL/6 mice to evaluate the efficacy of the novel strain on stress-related diseases.

## MODE FOR CARRYING OUT INVENTION

The terms used in this specification have been chosen to be as generic as possible in current common usage while considering their function in the invention, but they may vary depending on the intent or precedent of those skilled in the art, the emergence of new technologies, etc. In addition, in certain cases, terms have been chosen arbitrarily by the applicant, in which case their meaning will be described in detail in the description of the invention. Therefore, terms used in the invention should be defined based on the meaning of the term and the invention as a whole, rather than on the mere designation of the term.

Unless otherwise defined, all terms used herein, including technical or scientific terms, have the same meaning as commonly understood by one of ordinary skill in the art to which this invention belongs. Such terms, as defined in commonly used dictionaries, are to be construed to have meanings consistent with their contextual meaning in the relevant art and are not to be construed in an idealized or unduly formal sense unless expressly defined in this application.

The following describes the invention in more detail.

The present invention provides a strain of *Lactococcus lactis subsp. lactis* LB1022, deposited under accession number KCTC 15324BP.

The strain of *Lactococcus lactis subsp. lactis* LB1022 is characterized in that has a 16S rRNA represented by sequence number 1.

The strain can be utilized as a functional lactic acid bacteria due to its excellent acid tolerance, bile tolerance, and ability to adhere to intestinal epithelial cells.

Furthermore, the strain inhibits the production of at least one inflammatory factor selected from the group consisting of nitric oxide (NO), tumor necrosis factor-$\alpha$ (TNF-$\alpha$), interferon-$\gamma$ (IFN-$\gamma$), and interleukin-1$\beta$ (IL-1$\beta$), having an

anti-inflammatory effect by inhibiting gene expression of p65 or p50 in the Nuclear Factor Kappa B pathway. Thus, a composition comprising live bacteria, dead bacteria, or culture medium thereof of the strain as active ingredients can be utilized for the prevention, treatment, or amelioration of inflammatory diseases.

The present invention also provides a pharmaceutical composition for the prevention or treatment of inflammatory diseases, comprising as an active ingredient, live bacteria, dead bacteria, or a culture medium thereof of the strain. The present invention also provides a health functional food composition for the prevention or amelioration of inflammatory diseases comprising, as an active ingredient, live bacteria, dead bacteria, or a culture medium thereof of the strain.

The present invention also provides a health functional food composition for the prevention or amelioration of hangovers, comprising as an active ingredient, live bacteria, dead bacteria, or a culture medium thereof of the strain.

The present invention also provides a health functional food composition for the prevention or amelioration of liver function, comprising as an active ingredient, live bacteria, dead bacteria, or a culture medium thereof of the strain.

The present invention also provides a pharmaceutical composition for the prevention or treatment of allergic diseases, comprising as an active ingredient, live bacteria, dead bacteria or a culture medium thereof of the strain. The present invention also provides a health functional food composition for the prevention or amelioration of allergic diseases, comprising as an active ingredient, live bacteria, dead bacteria or a culture medium thereof of the strain.

The present invention also provides a pharmaceutical composition for the prevention or treatment of skin damage, comprising as an active ingredient, live bacteria, dead bacteria, or a culture medium thereof of the strain. The present invention also provides a cosmetic composition for preventing or improving skin damage, comprising as an active ingredient, live bacteria, dead bacteria or a culture medium thereof of the strain.

The present invention also provides a pharmaceutical composition for the prevention or treatment of obesity, comprising as an active ingredient, live bacteria, dead bacteria, or a culture medium thereof of the strain. The present invention also provides a health functional food composition for the prevention or amelioration of obesity, comprising as an active ingredient, live bacteria, dead bacteria or a culture medium thereof of the strain.

The present invention also provides a pharmaceutical composition for the prevention or treatment of diabetes, comprising as an active ingredient, live bacteria, dead bacteria or a culture medium thereof of the strain. The present invention also provides a health functional food composition for the prevention or amelioration of diabetes, comprising as an active ingredient, live bacteria, dead bacteria or a culture medium thereof of the strain.

The present invention also provides a pharmaceutical composition for the prevention or treatment of muscle loss, comprising as an active ingredient, live bacteria, dead bacteria or a culture medium thereof of the strain. The present invention also provides a health functional food composition for the prevention or amelioration of muscle loss, comprising as an active ingredient, live bacteria, dead bacteria, or a culture medium thereof of the strain.

The present invention also provides a health functional food composition for gastrointestinal protection, comprising as an active ingredient, live bacteria, dead bacteria or a culture medium thereof of the strain.

The pharmaceutical compositions of the present invention may be prepared in unit dose form by formulation in a pharmaceutically acceptable carrier, or in multi-dose containers, in accordance with methods readily practiced by a person of ordinary skill in the art to which the invention belongs.

The pharmaceutically acceptable carriers are those customarily used in the formulation, and include such as lactose, dextrose, sucrose, sorbitol, mannitol, starch, acacia gum, calcium phosphate, alginate, gelatin, calcium silicate, microcrystalline cellulose, polyvinylpyrrolidone, cellulose, water, syrup, methyl cellulose, methyl hydroxybenzoate, propyl hydroxybenzoate, talc, magnesium stearate, and mineral oils, but not limited thereto. In addition to the above ingredients, the pharmaceutical composition of the present invention may further comprise lubricants, wetting agents, sweeteners, flavoring agents, emulsifiers, suspending agents, preservatives, and the like.

In the present invention, the content of the additives in the pharmaceutical composition is not particularly limited and can be suitably adjusted within the range of content used in conventional formulations.

The pharmaceutical composition may be formulated in one or more topical forms selected from the group consisting of injectable formulations such as aqueous solutions, suspensions, emulsions, and the like, pills, capsules, granules, tablets, creams, gels, patchs, aerosols, ointments, plasters, lotions, liniments, pastes, and cataplasms.

The pharmaceutical compositions of the present invention may further comprise pharmaceutically acceptable carriers and diluents for formulation. Such pharmaceutically acceptable carriers and diluents include excipients such as starches, sugars, and mannitol etc., fillers and bulking agents such as calcium phosphate etc., cellulose derivatives such as carboxymethylcellulose, hydroxypropylcellulose etc., binders sunxh as gelatin, alginates, and polyvinyl pyrrolidone etc., lubricants such as talc, calcium stearate, hydrogenated castor oil, and polyethylene glycol etc., disintegrating agents such as povidone and crosspovidone, and surfactants such as polysorbates, cetyl alcohol, and glycerol etc., but not limited thereto. The above pharmaceutically acceptable carriers and diluents may be biologically and physiologically compatible with the subject. Examples of diluents include, but are not limited to, brines, aqueous buffers, solvents, and/or dispersion media.

The present invention can be used in general as a food product of conventional use.

The food compositions of the present invention can be used as health functional foods. The term "health functional food" means a food manufactured and processed using raw materials or ingredients that have a function useful to the human body in accordance with the Law on Health Functional Foods, and the term "functional" means consumed for the purpose of obtaining an effect useful for health purposes, such as regulating nutrients or physiological actions on the structure and function of the human body.

The food compositions of the present invention may include conventional food additives, and their suitability as "food additives" shall be determined according to the standards and criteria for such items in accordance with the General Principles and General Test Methods for Food Additives Code approved by the Ministry of Food and Drug Safety, unless otherwise specified.

The items listed in the above "Food Additives Code" include, for example, chemical compounds such as ketones, glycine, potassium citrate, nicotinic acid, cinnamic acid, etc., natural additives such as red pigments, licorice extract, crystalline cellulose, macrocolors, guar gum etc., mixed preparations such as monosodium L-glutamate preparations, noodle additive alkalis, preservative preparations, tar coloring preparations, etc.

The food compositions of the present invention can be prepared and processed in the form of tablets, capsules, powders, granules, liquids, pills, and the like.

For example, among the capsule-type health functional foods, hard capsules can be prepared by mixing the composition according to the present invention with excipients and other additives and filling conventional hard capsules, and soft capsules can be prepared by mixing the composition according to the present invention with excipients and other additives and filling a capsule base such as gelatin. The soft capsules may contain plasticizers such as glycerin or sorbitol etc., colorants, preservatives, etc. as needed.

The definitions of the terms excipients, binders, disintegrating agents, glossing agents, mating agents, flavoring agents, and the like are described in the literature known in the art and include those having the same or similar functions. The types of food products are not particularly limited and include all health functional foods in the conventional sense.

As used herein, the term "prevention" refers to any act of inhibiting or delaying a disease by administration of a composition according to the present invention. As used herein, the term "treatment" refers to any act of ameliorating or beneficially altering the condition of a disease by administration of a composition according to the invention. As used herein, the term "amelioration" refers to any act of making a bad condition of a disease better by administering or ingesting a composition of the present invention to a subject.

Hereinafter, the present invention will be described in detail with reference to experimental examples and embodiments for better understanding. However, the following experimental examples and embodiments are only illustrative of the present invention, and the scope of the present invention is not limited to the following experimental examples and embodiments. The experimental examples and embodiments of the present invention are provided to more fully explain the present invention to one of ordinary skill in the art.

**<Preparations> Control and comparison groups**

To evaluate the efficacy of the novel strain, *Lactococcus lactis subsp. lactis* LB1022, a type strain, *Lactococcus lactis subsp. lactis* KCTC 3769$^T$, was selected as a comparison group. Cells untreated with the inducer and experimental groups were defined as the control group. In the results of all experiments, the significance was expressed as the difference between the mean value and the T-test, ANOVA analysis value with the control group, where the significance * means $p < 0.05$, ** means $p < 0.005$, *** means $p < 0.0005$, **** means $p < 0.0001$, respectively.

**Example 1: Isolation and identification of novel functional lactic acid bacteria**

In order to discover various functional lactic acid bacteria, the process of isolating novel strains from fermented dairy products and identifying the species of the isolated novel strains was performed.

All dairy product samples were chopped and homogenized by agar plate dilution method using 10 ml of peptone solution (0.85% mass/vol) in 10-fold serial dilutions. The above diluted samples were smeared on Soybean-Casein Digest Agar (Tryptic Soy Agar-TSA; BD BBL) or Lactococcus selection medium (Alsan media) and incubated in a MIR-254-PK incubator (Panasonic, Osaka, Japan) at 30°C for 2-3 days. After incubation, the cultures were picked the white colonies formed and were subcultured several times on TSA for pure isolation. After the culture was completed, the cultures were suspended in 20% glycerol and stored in a -80°C freezer.

To identify the above isolates, the 16S ribosomal RNA gene (16S rDNA) was sequenced to determine their phylogenetic position. The 16S rDNA of the isolates was PCR amplified using universal primers for 16S rDNA, consisting of a forward primer represented by sequence number 2 (5'-AGAGTTTGATCCTGGCTCAG-3') and a reverse primer represented by sequence number 3 (5'-AAGGAGGTGATCCAGCC-3'). For further identification of the isolate, the specific genes of the *Lactococcus lactis* strain were analyzed by PCR amplification with a forward primer represented by sequence number 4 (5'-GTTGTATTAGCTAGTTGGTGAGGTAAAAA-3') and a reverse primer represented by sequence number 5 (5'-GTTGAGCCACTGCCTTTTAC-3'). The PCR was performed under the following conditions: 94°C for 1 minute for DNA denaturation, 50°C for 1 minute for DNA annealing, and 72°C for 1 minute 50 seconds for DNA extension, with 30 cycles of amplification.

The novel strain isolated by PCR analysis showed high similarity to type strain of the *Lactococcus lactis* in its 16S rRNA gene sequence, and the novel strain was identified as a *Lactococcus lactis subsp. lactis* strain by analyzing the specific genes of *Lactococcus lactis* strains. Furthermore, morphological analysis of the novel strain identified it as a Gram-positive coccus, identical to *Lactococcus lactis subsp. lactis.*

Therefore, the novel strain was named *Lactococcus lactis subsp. lactis* LB1022 and deposited at the Korean Collection for Type Cultures (KCTC) of the Korea Research Institute of Bioscience and Biotechnology on February 14, 2023 (deposit number: KCTC 15324BP).

**Example 2. Evaluation of bile and acid tolerance of a novel strain, *Lactococcus lactis subsp. lactis* LB1022**

To demonstrate that the novel strain, *Lactococcus lactis subsp. lactis* LB1022, is a functional lactic acid bacteria, the bile and acid tolerance of the novel strain were analyzed to evaluate its intestinal growth efficiency.

To evaluate the bile tolerance of the novel strain, Tryptic Soy Agar (TSA) medium containing 0.4% bile or Lactococcus selection medium (Alsan media) was used. The novel strain, *Lactococcus lactis subsp. lactis* LB1022, or the comparative strain *Lactococcus lactis subsp. lactis* KCTC 3769$^T$ was inoculated at a concentration of 1% v/v and incubated at 30°C for 6 h. Immediately after inoculation or at the end of the 6 h incubation, cultures were harvested and smeared on Tryptic Soy Agar (TSA) medium containing 0.4% bile or Lactococcus selection medium (Alsan media).

To evaluate the acid tolerance of the novel strain, Tryptic Soy Agar (TSA) medium with pH adjusted to 2.0 or Lactococcus selection medium (Alsan media) was used. The novel strain, *Lactococcus lactis subsp. lactis* LB1022, or the comparative strain Lactococcus lactis subsp. lactis KCTC 3769$^T$ was inoculated at a concentration of 1% (v/v) and incubated at 30°C for 2 h. Immediately after inoculation or at the end of the 2 h incubation, cultures were harvested and smeared on Tryptic Soy Agar (TSA) medium adjusted to pH 2.0 or Lactococcus selection medium (Alsan media).

The smeared strains were incubated at 30°C for 48 hours, and after incubation was completed, colonies were counted and analyzed for bile and acid tolerance.

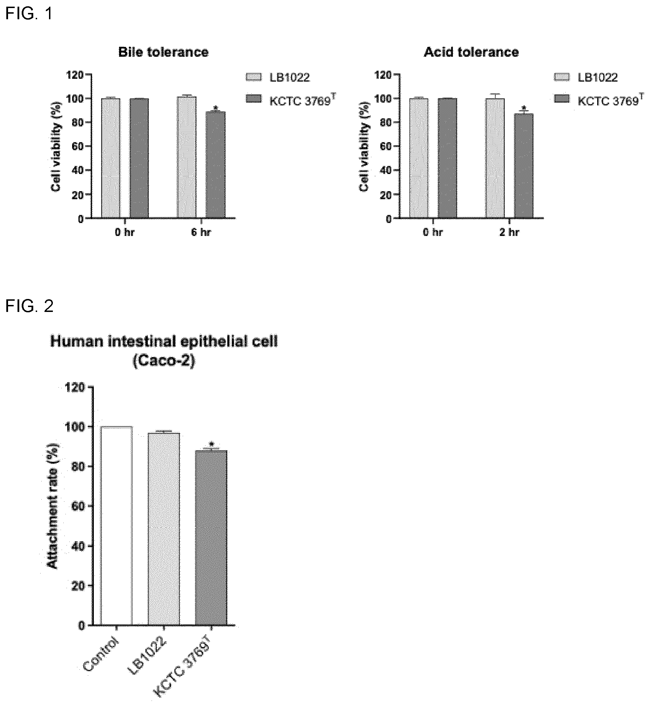

As shown in FIG. 1, after 6 hours of incubation in bile-containing medium, the viability of the control *Lactococcus lactis subsp. lactis* KCTC 3769$^T$ strain was slightly reduced from the initial one, while the survival rate of the novel strain, *Lactococcus lactis subsp. lactis* LB1022, has been shown to maintain its viability without decreasing. Furthermore, after 2 hours of incubation at acidic pH conditions, the viability of the control strain of *Lactococcus lactis subsp. lactis* KCTC 3769$^T$ decreased slightly from the initial level, but the viability of the novel strain, *Lactococcus lactis subsp. lactis* LB1022 was not decreased.

The above results demonstrate that the novel strain, *Lactococcus lactis subsp. lactis* LB1022, has superior bile and acid tolerance compared to the existing type strain, *Lactococcus lactis subsp. lactis* KCTC 3769$^T$, and suggests that it can be utilized as a useful lactic acid bacteria by growing in the intestinal environment.

**Example 3. Evaluation of intestinal adherence of the novel strain of *Lactococcus lactis subsp. lactis* LB1022**

To demonstrate that the novel strain, *Lactococcus lactis subsp. lactis* LB1022, is a functional lactic acid bacteria, the intestinal adherence was evaluated by co-culture with Caco-2 cells, a human intestinal epithelial cell line.

Minimum Essential Medium (MEM) medium containing 10% fetal bovine serum (FBS) and antibiotics (100 U/mL penicillin and 100 mg/mL streptomycin) was dispensed into T-75 flasks, Caco-2 cells were inoculated, and incubated at 37°C in 5% $CO_2$ until a cell monolayer was formed. The cultured Caco-2 cells were inoculated into 24-well plates at a concentration of 1 X 10$^5$ cells/ml and incubated until a cell monolayer was formed. The novel strain, *Lactococcus lactis subsp. lactis* LB1022, or the control, *Lactococcus lactis subsp. lactis* KCTC 3769$^T$, were diluted to a concentration of 1 X 10$^8$ in phosphate buffer saline (PBS) and applied to Caco-2 cell monolayers formed in 24-well plates, respectively. The plates were incubated at 37°C in 5% $CO_2$ for 2 hours, and unattached bacteria were removed by washing twice with PBS. Cells with each strain attached were obtained by treating with 0.25% trypsin-EDTA (ethylene-diamine-tetraacetic acid). Cells were serially diluted in PBS, then plated on Tryptic Soy Agar (TSA) medium or Lactococcus selective medium (Alsan media) and incubated for 48 hours at 30°C. After the incubation was completed, the colony count was measured and the ability of each strain to adhere to enterocytes was evaluated compared to the initial inoculum concentration.

As shown in FIG. 2, after 48 hours of co-incubation with Caco-2 cells, an enterocyte cell line, the rate of adherence of the control *Lactococcus lactis subsp. lactis* KCTC 3769$^T$ strain to Caco-2 cells was significantly reduced to 89±1.29%, while the adhesion rate of the novel strain, *Lactococcus lactis subsp. lactis* LB1022, to Caco-2 cells was barely reduced to 96±0.85%.

The above results demonstrate that the adhesion ability of the novel strain, *Lactococcus lactis subsp. lactis* LB1022 to the intestinal epithelium is superior to the existing type strain *Lactococcus lactis subsp. lactis* KCTC 3769$^T$, suggesting that it can be grown in the intestinal environment and utilized as a useful lactic acid bacteria.

**Example 4. Evaluation of Anti-inflammatory of the novel strain, *Lactococcus lactis subsp. lactis* LB1022**

To demonstrate that the novel strain, *Lactococcus lactis subsp. lactis* LB1022, is a functional lactic acid bacteria that exhibits anti-inflammatory ability, the anti-inflammatory effects of the strain were evaluated by incubating it with inflammation-induced macrophages and analyzing the levels of nitric oxide (NO) production, secretion of pro-inflammatory cytokines, and expression level of the pro-inflammatory NF-kB pathway.

RAW264.7 cells, a mouse macrophage cell line, were used as macrophages in which inflammation was induced. Dulbecco's Modified Eagle's Medium (DMEM) containing 10% fetal bovine serum (FBS) and antibiotics (20 $\mu$g/mL gentamicin) was dispensed into T-75 flasks, and RAW264.7 cells were inoculated and incubated at 37°C in 5% $CO_2$. The cultured RAW264.7 cells were plated in 24-well plates at a concentration of 1 X $10^5$ cells/ml and incubated until a cell monolayer was formed. To induce an inflammatory response, cells were treated with 1 $\mu$g/mL of lipopolysaccharide (LPS). The novel strain, *Lactococcus lactis subsp. lactis* LB1022 or the control, *Lactococcus lactis subsp. lactis* KCTC 3769$^T$ strain were diluted in phosphate buffer saline (PBS) to a concentration of 1 X $10^8$ cells/ml and applied onto RAW264.7 cell monolayers formed in 24-well plates, respectively.

**4-1. Evaluation of NO production levels**

Nitric oxide (NO), a pro-inflammatory substance induced by LPS in cells, is an important mediator of inflammatory diseases. To evaluate the anti-inflammatory effect of the strain, the level of nitric oxide (NO) production in the supernatant of inflammation-induced RAW264.7 cells was analyzed. RAW264.7 cells were centrifuged at 1,000 rpm for 5 minutes to obtain supernatants. The supernatant was mixed with Griess reagent in a 1:1 ratio and the absorbance at 540 nm was measured with a NanoQuant spectrophotometer (Infinite M200, Tecan, Switzerland).

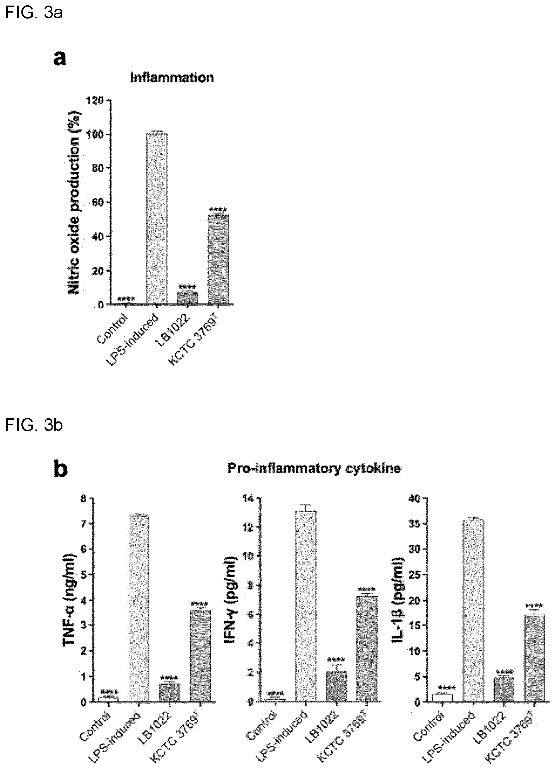

As shown in FIG. 3a, when the level of NO production in the LPS-induced inflammatory response was set at 100%, treatment with the control strain, *Lactococcus lactis subsp. lactis* KCTC 3769$^T$ reduced NO production rate by 52.5 $\pm$0.95%. On the other hand, treatment with the novel strain, *Lactococcus lactis subsp. lactis* LB1022, reduced NO production rate by 7.28$\pm$0.98%. The above results indicate that the inhibitory effect of the novel strain, *Lactococcus lactis subsp. lactis* LB1022, on the inflammatory response is significantly higher than that of the existing type strain, *Lactococcus lactis subsp. lactis* KCTC 3769$^T$, and demonstrate that the novel strain, *Lactococcus lactis subsp. lactis* LB1022, has superior anti-inflammatory effects.

**4-2. Evaluation of Cytokine secretion levels**

The secretion levels of pro-inflammatory cytokines were in response-induced RAW264.7 cells. The Quantikine immunoassay kit (R&D systems, Minneapolis, MN, USA) was used to measure the secretion levels of pro-inflammatory cytokines and was performed according to the manufacturer's manual, and The secretion levels of the pro-inflammatory cytokines TNF-$\alpha$, IFN-$\gamma$, and IL-1$\beta$ secreted by RAW264.7 cells were analyzed by measuring the absorbance at 540 nm with a NanoQuant spectrophotometer (Infinite M200, Tecan, Switzerland).

As shown in FIG. 3b, the secretion levels of TNF-$\alpha$, IFN-$\gamma$, and IL-1$\beta$ were 7.31$\pm$0.06 ng/ml, 13.12$\pm$0.41 pg/ml, and 35.74$\pm$0.49 pg/m, respectively, when the inflammatory response was induced by LPS. Treatment with the control strain, *Lactococcus lactis subsp. lactis* KCTC 3769$^T$, resulted in secretion levels of TNF-$\alpha$, IFN-$\gamma$, and IL-1$\beta$ of 3.6$\pm$0.06 ng/ml, 7.27$\pm$0.17 pg/ml, and 17.2$\pm$0.97 pg/ml, respectively. On the other hand, treatment with the novel strain, *Lactococcus lactis subsp. lactis* LB1022, significantly reduced the secretion levels of TNF-$\alpha$, IFN-$\gamma$, and IL-1$\beta$ to 0.71 $\pm$0.09 ng/ml, 2.06$\pm$0.46 pg/ml, and 4.88$\pm$0.31 pg/ml, respectively, compared to the control strain.

The above results demonstrate that the inhibitory effect of the novel strain, *Lactococcus lactis subsp. lactis* LB1022, on pro-inflammatory cytokine secretion is significantly better than that of the existing type strain, *Lactococcus lactis subsp. lactis* KCTC 3769$^T$, and demonstrate that the novel strain, *Lactococcus lactis subsp. lactis* LB1022, has superior anti-inflammatory effects.

**4-3. Evaluate of the expression levels of NF-kB pathway**

Activation of the Nuclear Factor Kappa B (NF-kB) pathway, an inflammatory immune response, leads to the expression of various proinflammatory cytokines, which can lead to the development of a number of inflammatory diseases. To evaluate the anti-inflammatory effect of the strain, the gene expression levels of NF-kB subunits, p65 and p50, were quantitatively analyzed by real-time PCR in RAW264.7 cells with induced inflammatory response.

RNA was extracted from RAW264.7 cells using Trizol (Sigma-Aldrich, Korea), and cDNA was synthesized using a cDNA synthesis kit (PrimeScript™ 1st strand cDNA Synthesis Kit, Takara, Japan). SYBR Green PCR master mix (Applied Biosystems, USA) was mixed with 2 $\mu$l of the above-synthesized cDNA, 0.5 $\mu$l of each primer set (10 $\mu$M) and RNase free water, and Real-time PCR was performed using an ABI 7500 real-time thermocycler (Biosystems, Foster City, CA, USA) machine at 50°C for 2 min and 95°C for 10 min. The expression levels were evaluated in comparison to the expression levels of the housekeeping gene GAPDH. The primers used for PCR are shown in Table 1 below.

**Table 1**

| Gene | Sequence Information (5' → 3') | | Sequence No. |
|---|---|---|---|
| p50 | Forward | cacaaggcagcaaatagacg | 6 |
| | Reverse | gagttaGCAGTgaggcacca | 7 |
| p65 | Forward | caggcgagaggagcacagatac | 8 |
| | Reverse | tcctttcctacaagctcgtgggggg | 9 |

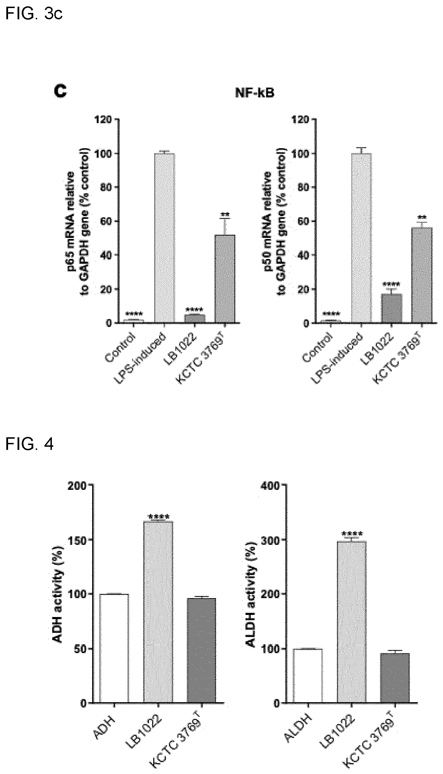

As shown in FIG. 3c, compared to the expression level of p65 (100 $\pm$ 1.44%) and p50 (100 $\pm$ 3.38%) in cells induced with an inflammatory response by LPS, the expression level of p65 in cells treated with the *Lactococcus lactis subsp. lactis* KCTC 3769$^T$ strain was 51.83 $\pm$ 9.73% and the expression level of p50 was 56.13 $\pm$ 3.14%. In contrast, the expression level of p65 in cells treated with a novel strain, *Lactococcus lactis subsp. lactis* LB1022, was 4.86 $\pm$ 0.41% and the expression level of p50 was 17.07 $\pm$ 3.04%. The expression level of p65 in normal cells was 1.88 $\pm$ 0.31% and the expression level of p50 was 1.45 $\pm$ 0.27%.

The above results demonstrate that the NF-kB pathway inhibition effect of the novel strain, Lactococcus lactis subsp. lactis LB1022 strain on inflammatory response is superior to that of the existing type strain, *Lactococcus lactis subsp. lactis* KCTC 3769$^T$, and prove that the novel strain, *Lactococcus lactis subsp.* lactis LB1022 strain has a superior anti-inflammatory effect.

**Example 5. Evaluation of hangover efficacy of the novel strain, *Lactococcus lactis* subsp. *lactis* LB1022**

To demonstrate that the novel strain, *Lactococcus lactis subsp. lactis* LB1022 is a functional lactic acid bacteria for hangover, alcohol and acetaldehyde degradation efficacy was evaluated by measuring alcohol dihydrogenase (ADH) and acetaldehyde dihydrogenase (ALDH) activities.

ADH activity was compared relative to the control via absorbance data, with ADH enzyme used as a control. A 3 ml of enzyme reaction solution was composed of 1.0 M Tris/HCl buffer (pH 8.8), 20 mM NAD$^+$, 0.2 M ethanol and distilled water. the strain of *Lactococcus lactis subsp. lactis* KCTC 3769$^T$ or the novel strain, *Lactococcus lactis subsp. lactis* LB1022 were treated and reacted for 5 min in a 30°C water bath, and the absorbance of the supernatant was measured at a wavelength of 340 nm in a NanoQuant spectrophotometer (Infinite M200, Tecan, Switzerland).

ALDH activity was compared relative to the control via absorbance data, and ALDH enzyme was used as a control. A 3 ml reaction solution was composed of 1.0 M Tris/HCl buffer (pH 8.0), 20 mM NAD$^+$, 3.0 M KCl, 0.33 M 2-mercaptoehanol, 1.0 M acetaldehyde, and distilled water. The strain of *Lactococcus lactis subsp. lactis* KCTC 3769$^T$ or the novel strain, *Lactococcus lactis subsp. lactis* LB1022, were treated and reacted for 5 min in a 30°C water bath. The absorbance of the supernatant was measured at a wavelength of 340 nm utilizing a NanoQuant spectrophotometer (Infinite M200, Tecan, Switzerland).

As shown in FIG. 4, when comparing the activity of the ADH enzyme, the treatment of *Lactococcus lactis subsp. lactis* KCTC 3769$^T$ strain was found to have an ADH activity of 95 $\pm$ 1.77%. On the other hand, the treatment of a novel strain, *Lactococcus lactis subsp. lactis* LB1022, was found to have an ADH activity of 166.77 $\pm$ 1.08%.

Furthermore, when comparing the activity of ALDH enzyme, the treatment of *Lactococcus lactis subsp. lactis* KCTC 3769$^T$ strain was found to have an ADH activity of 91.6 $\pm$ 5.71%. On the other hand, the treatment of *Lactococcus lactis subsp. lactis* LB1022, a novel strain, showed ADH activity of 296.95 $\pm$ 6.91%.

The above results demonstrate that the ADH and ALDH activity of the novel strain, *Lactococcus lactis subsp. lactis* LB1022 against hangover is superior to the existing type strain, *Lactococcus lactis subsp. lactis* KCTC 3769$^T$, and demonstrate that the novel strain, Lactococcus lactis subsp. lactis LB1022, has excellent hangover prevention and amelioration effects.

**Example 6. Evaluation of the efficacy of the novel strain, *Lactococcus lactis subsp. lactis* LB1022 in protecting liver function**

To demonstrate that the novel strain, *Lactococcus lactis subsp. lactis* LB1022, is a functional material for liver function protection, Sprague-Dawley (SD) rats were utilized to evaluate the efficacy through the analysis of indicators of alcoholic liver injury.

**6-1. Determination of Blood AST and ALT indicators**

Sprague-Dawley (SD) rats fasted for 16 hours were administered with *Lactococcus lactis subsp. lactis* KCTC 3769[T] strain or a novel strain, *Lactococcus lactis subsp. lactis* LB1022. After 30 minutes, 25% alcohol (10 ml/kg) was administered orally, and 24 hours after alcohol ingestion, Sprague-Dawley (SD) rats were sacrificed to obtain blood, and Aspartate aminotransferase (AST) and Alanine aminotransferase (ALT) levels were obtained by requesting data from Green Cross Lab Cell Co., Ltd., The blood AST and ALT levels were then compared with those of rats orally administered with alcohol alone.

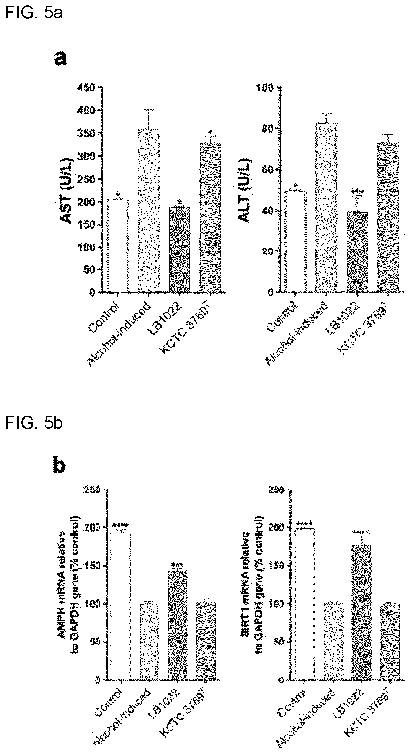

As shown in FIG. 5a, the blood AST level was found to be 327 ± 15.01 U/L in rats administered with *Lactococcus lactis subsp. lactis* KCTC 3769[T] strain compared to rats administered with alcohol alone (359 ± 41.96 U/L). On the other hand, the AST level in rats administered with the novel strain, *Lactococcus lactis subsp. lactis* LB1022, was found to be 189 ± 2.31 U/L. The AST level of normal rats was found to be 205 ± 2.89 U/L. In addition, the blood ALT level was found to be 72.5 ± 3.77 U/L in rats administered with Lactococcus lactis subsp. lactis KCTC 3769[T] strain compared to rats administered with alcohol alone (82.5 ± 4.70 U/L). In contrast, the ALT level of rats administered with the novel strain, *Lactococcus lactis subsp. lactis* LB1022 was found to be 39.5 ± 7.55 U/L. The ALT level of normal rats was found to be 49.5 ± 0.64 U/L.

**6-2. Determination of mRNA expression level of hepatoprotection-related factors**

Liver tissues from Sprague-Dawley (SD) rats were collected and tissue RNA was extracted with the RNeasy mini kit (Qiagen, Germany). The extracted tissue RNA was used to synthesize cDNA using a cDNA synthesis kit (Takara, Japan). Real-time PCR quantitative analysis was performed using an ABI 7500 real-time thermocycler (Biosystems, Foster City, CA, USA) using the same method as in Example 4-3 to determine the expression level of hepatoprotection-related factors (AMPK, SIRT1). The primer information used for PCR is shown in Table 2 below.

**Table 2**

| Gene | Sequence information (5' → 3') | | Sequence No. |
|---|---|---|---|
| AMPK | Forward | agagctgctcgagccaccaatgta | 10 |
| | Reverse | caccaatctgatggcccttcttcac | 11 |
| SIRT | Forward | gcgcttctattcctgctgctgctcacgg | 12 |
| | Reverse | gtggcatcctggacccacttcttc | 13 |
| GAPDH | Forward | tttgcctagaatcccccccacag | 14 |
| | Reverse | taaggagcccagaaaacagc | 15 |

As shown in FIG. 5b, compared with the expression level of hepatoprotection-related factors AMPK (100 ± 3.19%) and SIRT1 (100 ± 1.87%) in rats administered with alcohol alone, the expression level of AMPK in rats administered with *Lactococcus lactis subsp. lactis* KCTC 3769[T] strain was 101.94 ± 3.16%, and the expression level of SIRT1 was 99.37 ± 1.65%. In contrast, rats administered with the novel strain, *Lactococcus lactis subsp. lactis* LB1022 had the AMPK expression level of 143.48 ± 2.81% and the SIRT1 expression level of 177.09 ± 11.56%. The expression level of AMPK in normal rats was 193.22±4.15%, and the expression level of SIRT1 was 198.55±0.93%.

The above results demonstrated that the effect of AMPK/SIRT1 pathway activity of the novel strain, *Lactococcus lactis subsp. lactis* LB1022 on hepatoprotection was significantly better than that of the existing type strain, *Lactococcus lactis subsp. lactis* KCTC 3769[T], and demonstrate that the novel strain, *Lactococcus lactis subsp. lactis* LB1022, has superior hepatoprotective effects.

**Example 7. Evaluation of the efficacy of the novel strain, *Lactococcus lactis subsp. lactis* LB1022 against allergic diseases**

To demonstrate that the novel strain, *Lactococcus lactis subsp. lactis* LB1022, is a functional material for allergic diseases, female BALB/c mice were utilized to evaluate its effect on allergic dermatitis and asthma.

**7-1. Determination of serum IgE level and mRNA expression level of factors involved in allergic dermatitis**

Female BALB/c mice were sensitized intraperitoneally with a mixture of 50 ug/ml of ovalbumin and 50 ug/ml of Alum (Sigma-Aldrich, USA), an immunostimulant, at weeks 1, 3, 5, and 7, and allergic disease was induced by periodic exposure of mice to a nebulized 2% ovalbumin (w/v) dissolved in PBS in the respiratory tract. Mice were then orally administered with either *Lactococcus lactis subsp. lactis* KCTC 3769$^T$ strain or a novel strain, *Lactococcus lactis subsp. lactis* LB1022 for 8 weeks. Blood was obtained from sacrificed BALB/c mice at the end of the study, stored at 4°C for 1 hour and centrifuged at 5,000 x g for 1 hour to isolate serum. Serum IgE assay was performed according to the manufacturer's instructions of the ELISA kit (R&D systems, USA) and absorbance was measured at 450 nm wavelength using a NanoQuant spectrophotometer (Infinite M200, Tecan, Switzerland).

In addition, RNA was extracted from the obtained skin tissue using the RNeasy mini kit (Qiagen, Germany). The extracted skin tissue RNA was used to synthesize cDNA using the cDNA synthesis kit (Takara, Japan). Real-time PCR quantitative analysis was performed using an ABI 7500 real-time thermocycler (Biosystems, Foster City, CA, USA) using the same method as in Example 4-3 to determine the expression level of allergic dermatitis-related factors (TARC and Eotaxin). The primer information used for PCR is shown in Table 3 below.

**Table 3**

| Gene | Sequence information (5' → 3') | | Sequence No. |
|---|---|---|---|
| TARC | Forward | agagctgctcgagccaccaatgta | 16 |
| | Reverse | caccaatctgatggcccttcttcac | 17 |
| Eotaxin | Forward | gcgcttctattcctgctgctgctcacgg | 18 |
| | Reverse | gtggcatcctggacccacttcttc | 19 |

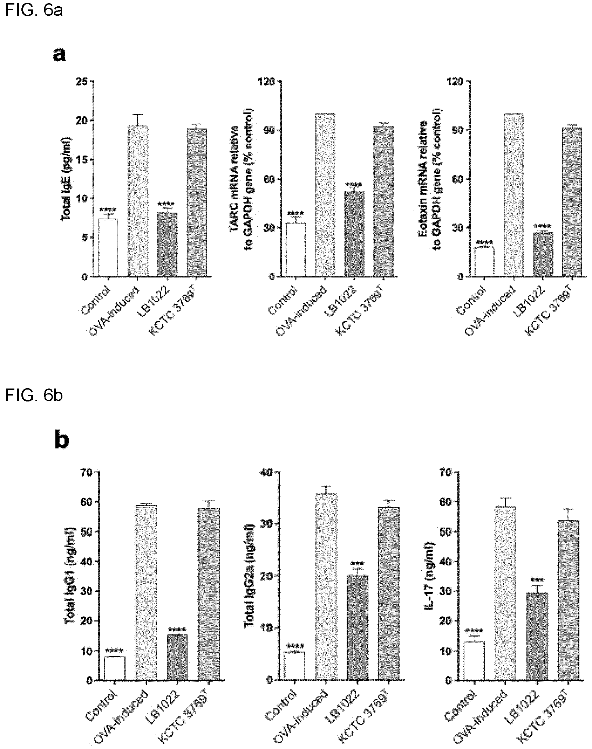

As shown in FIG. 6a, compared to the serum IgE level of mice with induced allergic disease (19.34 ± 1.39 ng/ml), the serum IgE level of mice administered with *Lactococcus lactis subsp. lactis* KCTC 3769$^T$ strain was 18.96 ± 0.59 ng/ml. In contrast, mice administered with the novel strain, *Lactococcus lactis subsp. lactis* LB1022 had the serum IgE level of 8.19 ± 0.54 ng/ml. The serum IgE level of normal mice was 7.38 ± 0.63 ng/ml.

Furthermore, compared to the expression level of TARC (100%) and Eotaxin (100%), which are factors involved in dermatitis, in mice with induced allergic disease, the expression level of TARC in mice administered with *Lactococcus lactis subsp. lactis* KCTC 3769$^T$ strain was 92.22 ± 2.19% and the expression level of Eotaxin was 91.16 ± 2.06%. In contrast, mice administered with a novel strain, *Lactococcus lactis subsp. lactis* LB1022, had TARC expression level of 52.26 ± 2.51% and Eotaxin expression level of 26.94 ± 1.12%. The expression level of TARC in normal mice was 32.97 ± 3.81%, and the expression level of Eotaxin was 18.06 ± 0.34%.

## 7-2. Determination of serum IgG1, IgG2a levels and cytokine expression levels in bronchoalveolar lavage fluid (BALF)

Blood was obtained from BALB/c mice sacrificed at the end of the study, stored at 4°C for 1 hour and centrifuged at 5,000 x g for 1 hour to separate serum. Serum IgG1, IgG2a assays were performed according to the manufacturer's instructions for the ELISA kit (Abcam, UK) and absorbance was measured at a wavelength of 450 nm using a NanoQuant spectrophotometer (Infinite M200, Tecan, Switzerland).

To obtain alveolar fluid, the skin on the neck and thorax of the mice was incised to expose the bronchi and lungs, and the alveoli were flushed with PBS through the bronchus at 0.5 ml per time for three times. The recovered alveolar fluid was centrifuged at 4°C, 1,000 rpm for 10 min, and the supernatant was obtained and analyzed for cytokines according to the manufacturer's instructions of the ELISA kit (R&D systems, USA).

As shown in FIG. 6b, compared with the serum IgG1 level of mice with induced allergic asthma (58.85 ± 0.47 ng/ml), the serum IgG1 level of mice administered with *Lactococcus lactis subsp. lactis* KCTC 3769$^T$ strain was 57.77 ± 2.52 ng/ml. In contrast, mice administered with the novel strain, *Lactococcus lactis subsp. lactis* LB1022 had the serum IgG1 level of 15.35 ± 0.03 ng/ml. Normal mice had the serum IgG1 level of 8.15 ± 0.05 ng/ml.

Furthermore, compared to the serum IgG2a level of mice with induced allergic asthma (35.88 ± 1.37 ng/ml), the serum IgG2a level of mice administered with *Lactococcus lactis subsp. lactis* KCTC 3769$^T$ strain was 33.26 ± 1.28 ng/ml. In contrast, mice administered with the novel strain, *Lactococcus lactis subsp. lactis* LB1022 had the serum IgG2a level of 20.06 ± 1.29 ng/ml. Normal mice had the serum IgG2a level of 5.41 ± 0.17 ng/ml.

Compared to the level of IL-17 in the alveoli of mice with induced allergic asthma (58.36 ± 2.82 ng/ml), the level of IL-17 in the alveoli of rats administered with *Lactococcus lactis subsp. lactis* KCTC 3769$^T$ strain was 53.69 ± 3.81 ng/ml. In contrast, mice administered with the novel strain, *Lactococcus lactis subsp. lactis* LB1022, had the alveolar IL-17 level of 29.47 ± 2.43 ng/ml. The level of IL-17 in the alveoli of normal mice was 13.21 ± 1.78 ng/ml.

The above results demonstrate that the novel strain, *Lactococcus lactis subsp. lactis* LB1022, has superior prevention and amelioration effects on allergic diseases compared to the existing type strain, *Lactococcus lactis subsp. lactis* KCTC 3769[T].

### Example 8. Evaluation of the efficacy of the novel strain, *Lactococcus lactis subsp. lactis* LB1022 against skin damage

To demonstrate that the novel strain, *Lactococcus lactis subsp. lactis* LB1022, is a functional material for skin damage, human skin epithelial cells were utilized to evaluate the skin regeneration efficacy.

HaCaT cells, a human skin epithelial cell line, were used as the cells in which skin damage was induced. Dulbecco's Modified Eagle's Medium (DMEM) medium containing 10% fetal bovine serum (FBS) and antibiotics (100 units/ml penicillin; Penicilin, 100 ug/ml streptomycin; Streptomicin) was dispensed into T-75 flasks and HaCaT cells were inoculated and cultured at 37°C in 5% $CO_2$. The cultured HaCaT cells were inoculated into 6-well plates and incubated until a cell monolayer was formed. When the cell monolayer was close to 100%, the cells were scratched with a tip to induce skin damage and then treated with either *Lactococcus lactis subsp. lactis* KCTC 3769[T] strain or the novel strain, *Lactococcus lactis subsp. lactis* LB1022. The changes of the cells over time were photographed under a microscope, and the scratch size was measured using the ImageJ software program (http://imagej.nih.gov/ij/index.html) to quantify the degree of skin re-epithelialization.

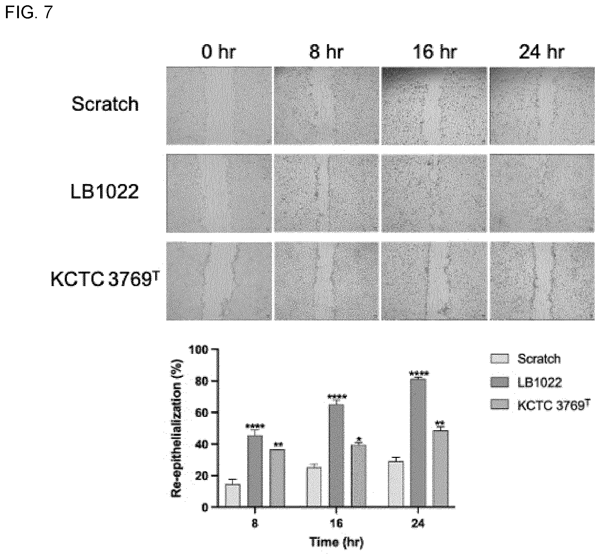

As shown in FIG. 7, compared to the skin regeneration rate of HaCaT cells (14.62 $\pm$ 3.08%) 8 hours after skin damage by scratch, the skin regeneration rate of cells treated with *Lactococcus lactis subsp. lactis* KCTC 3769[T] strain was 36.57 $\pm$ 0.03%. In contrast, the skin regeneration rate of cells treated with the novel strain, *Lactococcus lactis subsp. lactis* LB1022, was 45.58 $\pm$ 3.21%.

Compared to the skin regeneration rate of HaCaT cells 16 hours after skin damage (25.05 $\pm$ 2.19%), the skin regeneration rate of cells treated with *Lactococcus lactis subsp. lactis* KCTC 3769[T] strain was 39.94 $\pm$ 1.35%. In contrast, the skin regeneration rate of cells treated with a novel strain, *Lactococcus lactis subsp. lactis* LB1022, was 64.96 $\pm$ 2.99%.

Compared to the skin regeneration rate of HaCaT cells 24 hours after skin damage (29.27 $\pm$ 2.27%), the skin regeneration rate of cells treated with *Lactococcus lactis subsp. lactis* KCTC 3769[T] strain was 48.58 $\pm$ 2.38%. On the other hand, the skin regeneration rate of cells treated with the novel strain, *Lactococcus lactis subsp. lactis* LB1022, was 81.49 $\pm$ 0.87%.

The above results prove that the novel strain, *Lactococcus lactis subsp. lactis* LB1022 has a superior skin regeneration (wound healing) effect compared to the existing type strain, *Lactococcus lactis subsp. lactis* KCTC 3769[T] on skin damage.

### Example 9. Evaluation of the efficacy of the novel strain, *Lactococcus lactis subsp. lactis* LB1022 against obesity

To demonstrate that the novel strain, *Lactococcus lactis subsp. lactis* LB1022, is a functional material for obesity, male C57BL/6 mice were utilized to evaluate its efficacy in reducing body fat.

Male C57BL/6 mice were fed a 60% kcal% fat diet (Research Diets, USA) to induce high-fat diet-induced obesity for 10 weeks and orally administered with either *Lactococcus lactis subsp. lactis* KCTC 3769[T] strain or a novel strain, *Lactococcus lactis subsp. lactis* LB1022. At the end of the study, the body composition of C57BL/6 mice was analyzed by dual-energy X-ray absorptiometry using an Inalyzer (Medikors Inc, Korea). The mice were scanned whole body and the scan images and fat mass levels were measured using the Inalyzer software.

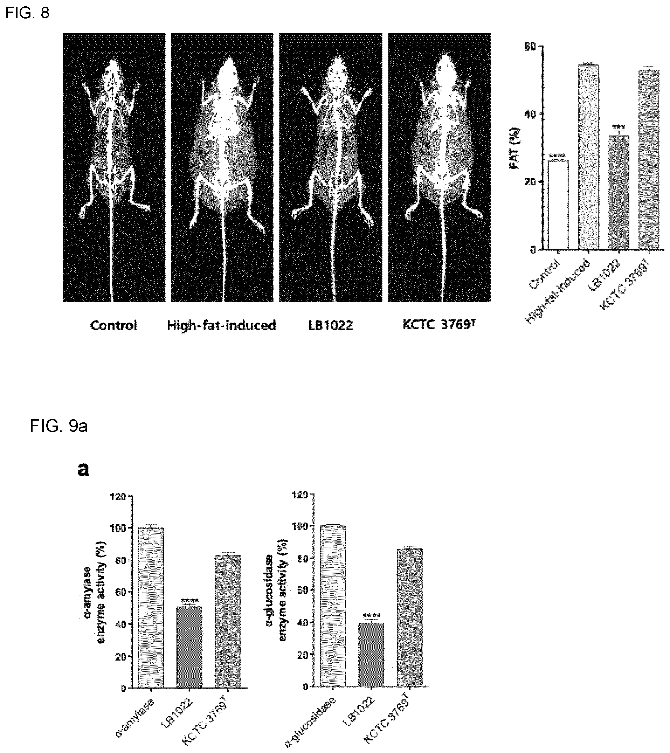

As shown in FIG. 8, compared to the fat-to-body weight ratio (54.59 $\pm$ 0.32%) of mice induced obesity by a high-fat diet for 12 weeks, mice administrated with *Lactococcus lactis subsp. lactis* KCTC 3769[T] strain had the fat-to-body weight ratio of 52.96 $\pm$ 0.97%. In contrast, mice administrated with the novel strain, *Lactococcus lactis subsp. lactis* LB1022 had the fat-to-body weight ratio of 33.59 $\pm$ 1.43%. The of normal mice was 26.21 $\pm$ 0.41%.

The above results demonstrate that the novel strain, *Lactococcus lactis subsp. lactis* LB1022 has a superior effect of preventing and improving obesity compared to the existing type strain of *Lactococcus lactis subsp. lactis* KCTC 3769[T].

### Example 10. Evaluation of the efficacy of the novel strain, *Lactococcus lactis subsp. lactis* LB1022 on diabetes

To demonstrate that the novel strain, *Lactococcus lactis subsp. lactis* LB1022, is a functional material for diabetes, its efficacy was evaluated by analyzing the inhibitory effect on glycolytic enzyme activity and the indicators involved in blood glucose reduction using male C57BL/6 mice.

**10-1. Confirmation of inhibition of glycolytic enzyme activity**

To confirm the inhibitory effect on α-amylase enzyme activity, 11 U/ml of α-amylase enzyme was mixed with *Lactococcus lactis subsp. lactis* KCTC 3769[T] strain or the novel strain, *Lactococcus lactis subsp. lactis* LB1022 and incubated at room temperature (25°C) for 10 minutes. After the incubation was completed, 1% starch as the substrate was added and incubated again at room temperature (25°C) for 10 minutes. DNS chromogenic reagent was prepared by adding 45 mM of 3,5-dinitrosalicylic acid in 30% sodium potassium tartrate, added to the above incubation solution, and heated in 100°C boiling water for 5 minutes to develop color. The reaction solution was cooled and the absorbance was measured at a wavelength of 540 nm using a NanoQuant spectrophotometer (Infinite M200, Tecan, Switzerland).

To determine the inhibitory effect on α-glucosidase enzyme activity, 1 U/ml of α-glucosidase enzyme was mixed with *Lactococcus lactis subsp. lactis* KCTC 3769[T] strain or the novel strain, *Lactococcus lactis subsp. lactis* LB1022, and incubated at 37 °C for 10 min. After completion of incubation, 3 mM of p-nitrophenyl α-D-glucopyranoside (pNPG) was added and incubated at 37°C for 20 minutes. The reaction was stopped by treating with 1 M of sodium carbonate and the absorbance was measured at a wavelength of 405 nm.

As shown in FIG. 9a, compared to the activity level of α-amylase enzyme (100 ± 1.78%), the activity level of α-amylase enzyme by *Lactococcus lactis subsp. lactis* KCTC 3769[T] strain treatment was 82.96 ± 1.61 ng/ml. On the other hand, the activity level of α-amylase enzyme by the treatment of the novel strain, *Lactococcus lactis subsp. lactis* LB1022, was 51.05 ± 1.32 ng/ml.

Compared with the activity level of α-glucosidase enzyme (100 ± 0.73%), the activity level of α-glucosidase enzyme by treatment with *Lactococcus lactis subsp. lactis* KCTC 3769[T] strain was 85.57 ± 1.63 ng/ml. On the other hand, the activity of α-glucosidase enzyme by treatment with the novel strain, *Lactococcus lactis subsp. lactis* LB1022 was 339.64 ± 2.17 ng/ml.

**10-2. Confirmation of blood glucose reduction effect in diabetes-induced mice**

Male C57BL/6 mice were fed a 60% kcal% fat diet (Research Diets, USA) for 10 weeks to induce type 2 diabetes by high-fat diet and orally administered *Lactococcus lactis subsp. lactis* KCTC 3769[T] strain or a novel strain, *Lactococcus lactis subsp. lactis* LB1022. Fasting insulin concentration (uU/L) and fasting glucose concentration (mg/dL) data were obtained through blood analysis at the end of the study, and the following equation was applied to calculate Homeostasis model assessment for insulin resistance (HOMA-IR) based on the results.

HOMA-IR = [fasting insulin concentration (uU/L)] × [fasting glucose concentration (mg/dL)]/405

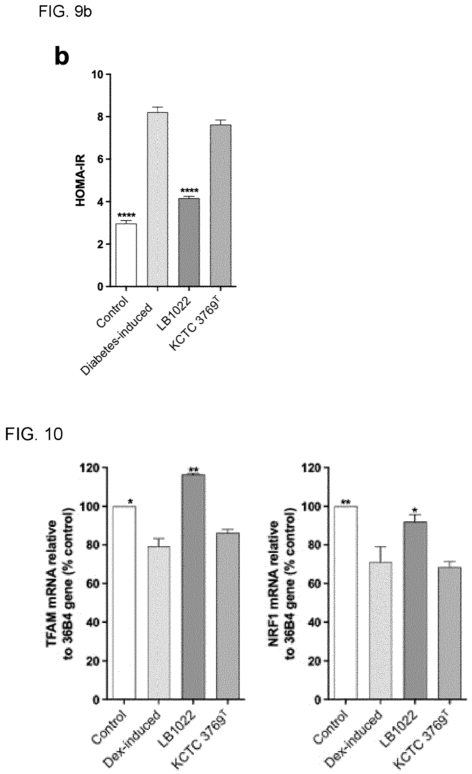

As shown in FIG. 9b, compared to HOMA-IR levels (8.21 ± 0.23) in mice induced with type 2 diabetes for 12 weeks, mice administrated with *Lactococcus lactis subsp. lactis* KCTC 3769[T] strain had the HOMA-IR level of 7.63 ± 0.21. In contrast, mice administrated with the novel strain, *Lactococcus lactis subsp. lactis* LB1022, had the HOMA-IR value of 4.16 ± 0.08%. The fat-to-body weight ratio of normal mice was 2.96 ± 0.14.

The above results demonstrate that the novel strain, *Lactococcus lactis subsp. lactis* LB1022, has superior diabetes prevention and amelioration effects compared to the existing type strain, *Lactococcus lactis subsp. lactis* KCTC 3769[T], on diabetes.

**Example 11. Evaluation of the efficacy against muscle loss of the novel strain, *Lactococcus lactis subsp. lactis* LB1022**

To demonstrate that the novel strain, Lactococcus lactis subsp. lactis LB1022, is a functional material against muscle loss, its efficacy was evaluated by analyzing indicators involved in muscle differentiation using muscle cells.

C2C12 cells, a mouse skeletal muscle cell line, were used for muscle differentiation. Dulbecco's Modified Eagle's Medium (DMEM) containing 10% fetal bovine serum (FBS) and antibiotics (100 U/mL penicillin and 100 mg/mL streptomycin) was dispensed into T-75 flasks, C2C12 cells were inoculated, and cultured at 37°C in 5% $CO_2$ until the cells reached 70-80% confluence. The cultured C2C12 cells were inoculated in 12-well plates at a concentration of 5 X 10[5] cells/ml and incubated until a monolayer of cells was formed. To induce differentiation of C2C12 cells, DMEM medium (Lonza, USA) containing 2% horse-serum (Gibco, USA) was added and cultured. C2C12 cells were treated with 1uM of dexamethasone (Sigma-Aldrich, USA) to induce muscle loss. The medium was replaced with fresh medium of the same composition every 2 days. C2C12 cells were treated with *Lactococcus lactis subsp. lactis* KCTC 3769[T] strain or the novel strain, *Lactococcus lactis subsp. lactis* LB1022, on day 6 of C2C12 cell differentiation and allowed to respond for 24 hours.

RNA was extracted from C2C12 cells using Trizol (Sigma-Aldrich, Korea), and cDNA was synthesized using a cDNA synthesis kit (PrimeScript™ 1st strand cDNA Synthesis Kit, Takara, Japan). Real-time PCR quantitative analysis was performed using an ABI 7500 real-time thermocycler (Biosystems, Foster City, CA, USA) using the same method as in Example 4-3 to determine the expression level of mitochondrial biosynthesis-related factors (TFAM, NRF1) for muscle differentiation. The primer information used for PCR is shown in Table 4 below.

**Table 4**

| Gene | Sequence information (5' → 3') | | Sequence No. |
|---|---|---|---|
| TFAM | Forward | gggtatggggagaaggaggccc | 20 |
| | Reverse | tccctgagccgaatcatcct | 21 |
| NRF1 | Forward | agggccggtgaaatgaccatc | 22 |
| | Reverse | cggcagcttcactgttgagg | 23 |

As shown in FIG. 10, when compared with the expression levels of the mitochondrial biosynthesis-related factors TFAM (79.19 ± 4.06 %) and NRF1 (71.09 ± 7.88 %) in C2C12 cells in which muscle loss was induced by dexamethasone during the differentiation process, the TFAM expression level and the NRF1 expression level in cells treated with the *Lactococcus lactis subsp. lactis* KCTC 3769[T] strain were 86.21 ± 1.85 % and 68.41 ± 3.01 %, respectively. In contrast, the TFAM expression level and the NRF1 expression level in cells treated with the novel strain, *Lactococcus lactis subsp. lactis* LB1022 were 116.33 ± 0.63% and 91.96 ± 3.78%, respectively. The expression level of TFAM and NRF1 in normal cells was 100%.

The above results demonstrate that the novel strain, *Lactococcus lactis subsp. lactis* LB1022 has a superior effect of preventing and improving muscle loss compared to the existing type strain *Lactococcus lactis subsp. lactis* KCTC 3769[T].

## Example 12. Evaluation of the efficacy of the novel strain, *Lactococcus lactis subsp. lactis* LB1022 on gastrointestinal protection

To demonstrate that the novel strain, *Lactococcus lactis subsp. lactis* LB1022, is a functional material for gastrointestinal protection, gastrointestinal epithelial cells were utilized to evaluate its efficacy through the analysis of indicators involved in gastrointestinal protection.

AGS cells, a human-derived gastrointestinal epithelial cell line commonly used for gastrointestinal protection studies, were used. RPMI 1640 (Gibco, USA) medium containing 10% fetal bovine serum (FBS; fetal bovine serum) and antibiotics (100 U/mL penicillin and 100 mg/mL streptomycin) was dispensed into T-75 flasks, and AGS cells were inoculated and cultured at 37°C under 5% $CO_2$. The cultured AGS cells were inoculated into 12-well plates at a concentration of 5 X $10^4$ cells/ml and incubated until a cell monolayer was formed. AGS cells were treated with 1 X $10^{48}$ CFU/ml of Helicobacter pylori to induce damage. AGS cells were treated with *Lactococcus lactis subsp. lactis* KCTC 3769[T] strain or the novel strain, *Lactococcus lactis subsp. lactis* LB1022, and incubated for 2 hours.

RNA was extracted from AGS cells using Trizol (Sigma-Aldrich, Korea), and cDNA was synthesized using a cDNA synthesis kit (PrimeScript™ 1st strand cDNA Synthesis Kit, Takara, Japan). Real-time PCR quantitative analysis was performed using the same method as in Example 4-3 using an ABI 7500 real-time thermocycler (Biosystems, Foster City, CA, USA) to determine the expression level of factors involved in pathogen interaction (Integrin α5 and Integrin β1). The primer information used for PCR is shown in Table 5 below.

**Table 5**

| Gene | Sequence information (5' → 3') | | Sequence No. |
|---|---|---|---|
| Integrin α5 | Forward | ctcagtgggagtttttaccggc | 24 |
| | Reverse | aggtagacagcaccaccaccctg | 25 |
| Integrin β1 | Forward | gaggggttgccctccaga | 26 |
| | Reverse | gcttgagcttctctctgctgtt | 27 |

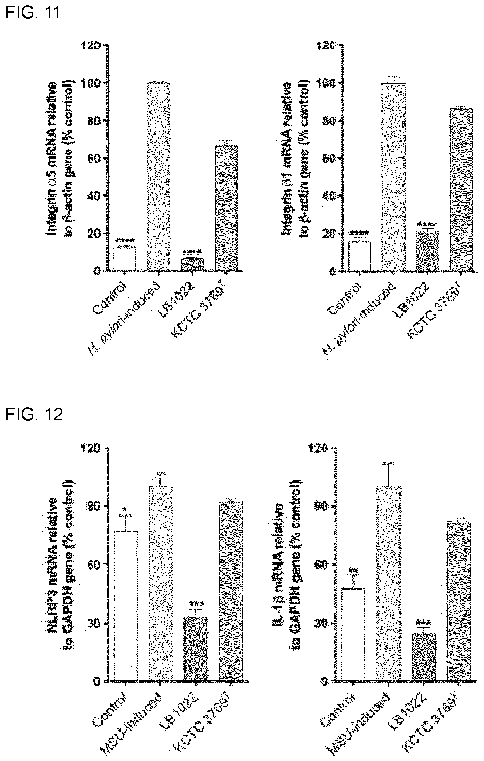

As shown in FIG. 11, compared with the expression level of Integrin α5 (100 ± 0.53%) and Integrin β1 (100 ± 3.35%), factors involved in pathogen interaction, in AGS cells whose cell damage was induced by Helicobacter pylori, the expression level of Integrin α5 in cells treated with *Lactococcus lactis subsp. lactis* KCTC 3769[T] strain was 66.39 ± 3.18% and the expression level of Integrin β1 was 86.39 ± 1.14%. In contrast, the expression level of Integrin α5 in cells treated with the novel strain, *Lactococcus lactis subsp. lactis* LB1022, was 6.95 ± 0.22% and the expression level of Integrin β1

was $20.72 \pm 1.81\%$. Integrin $\alpha 5$ expression level in normal cells was $12.65 \pm 0.71\%$, and Integrin $\beta 1$ expression level was $15.78 \pm 2.35\%$.

The above results demonstrate that the novel strain *Lactococcus lactis subsp. lactis* LB1022 has a superior gastrointestinal protective effect compared to the existing type strain, *Lactococcus lactis subsp. lactis* KCTC 3769$^T$ against gastrointestinal damage caused by pathogens including Helicobacter pylori strains.

**Example 13. Evaluation of the efficacy of the novel strain, Lactococcus lactis subsp. lactis LB1022 against gout**

To demonstrate that the novel strain, *Lactococcus lactis subsp. lactis* LB1022, is a functional material for gout, human-derived macrophages were utilized to evaluate its efficacy by analyzing indicators involved in gouty inflammation.

THP-1 cells, a monocytic cell line, were used for the gout study. RPMI 1640 (Gibco, USA) medium containing 10% fetal bovine serum (FBS) and antibiotics (100 U/mL penicillin and 100 mg/mL streptomycin) was dispensed into T-75 flasks, THP-1 cells were inoculated, and were cultured at 37°C in 5% $CO_2$ until they reached 70-80% confluence. The cultured THP-1 cells were inoculated into 12-well plates at a concentration of $5 \times 10^5$ cells/ml, treated with 250 ng/ml of phorbol 12-myristate 13-acetate (PMA) for 3 hours and replaced with fresh medium to differentiate the cells. To induce gouty cell inflammation, cells were treated with 100 $\mu$g/ml of MSU (Invivogen, USA) and reacted for 24 h with either *Lactococcus lactis subsp. lactis* KCTC 3769$^T$ strain or the novel strain, *Lactococcus lactis subsp. lactis* LB1022.

RNA was extracted from THP-1 cells using Trizol (Sigma-Aldrich, Korea), and cDNA was synthesized using a cDNA synthesis kit (PrimeScript™ 1st strand cDNA Synthesis Kit, Takara, Japan). Real-time PCR quantitative analysis was performed using an ABI 7500 real-time thermocycler (Biosystems, Foster City, CA, USA) to determine the expression level of inflammatory inflammasome factors (NLRP3 and IL-1$\beta$) using the same method as in Example 4-3. The primer information used for PCR is shown in Table 6 below.

**Table 6**

| Gene | Sequence information (5' → 3') | | Sequence No. |
|---|---|---|---|
| NLRP3 | Forward | atcagtattgagcacccagccatt | 28 |
| | Reverse | agagtgttgccctcgcaggtaaag | 29 |
| IL-1$\beta$ | Forward | cctgtcctgcgtgttgaaaga | 30 |
| | Reverse | gggaaactggggcgaactcaaa | 31 |

As shown in FIG. 12, compared with the expression level of inflammatory inflammasome factors NLRP3 ($100 \pm 6.50\%$) and IL-1$\beta$ ($100 \pm 12.07\%$) in THP-1 cells with induced gouty inflammation, the expression level of NLRP3 in cells treated with *Lactococcus lactis subsp. lactis* KCTC 3769$^T$ strain was $92.32 \pm 1.54\%$ and the expression level of IL-1$\beta$ was $81.64 \pm 2.18\%$. In contrast, the NLRP3 expression level in cells treated with the novel strain, *Lactococcus lactis subsp. lactis* LB1022 was $33.28 \pm 3.91\%$ and the expression level of IL-1$\beta$ was $24.79 \pm 2.77\%$. The expression level of NLRP3 in normal cells was $77.29 \pm 7.98\%$, and the expression level of IL-1$\beta$ was $47.91 \pm 6.95\%$.

The above results demonstrate that the novel strain, *Lactococcus lactis subsp. lactis* LB1022 has a superior prevention and improvement effect on gout compared to the existing type strain, *Lactococcus lactis subsp. lactis* KCTC 3769$^T$

**Example 14. Evaluation of the efficacy of the novel strain, *Lactococcus lactis subsp. lactis* LB1022 against stress-related diseases**

To demonstrate that the novel strain, *Lactococcus lactis subsp. lactis* LB1022, is a functional material for stress-related diseases, male C57BL/6 mice were utilized to evaluate its effect on reducing corticosterone and decreasing the expression level of stress-related factors in adipose.

Male C57BL/6 mice were subjected to cold stress by placing them on ice for 1 hour daily without food and water for 12 weeks, and after completion, stress related diabetes was induced in a dietary environment with a 60% kcal% fat diet (Research Diets, USA). During the stress related diabetes induction period, mice were orally administrated with either *Lactococcus lactis subsp. lactis* KCTC 3769$^T$ strain or the novel strain, *Lactococcus lactis subsp. lactis* LB1022. At the end of the study, blood was obtained from the mice and serum was isolated. Serum corticosterone assay was performed according to the manufacturer's instructions of the ELISA kit (R&D systems, USA) and absorbance was measured at a wavelength of 405 nm using a NanoQuant spectrophotometer (Infinite M200, Tecan, Switzerland).

Adipose tissue from C57BL/6 mice was collected and tissue RNA was extracted with the RNeasy mini kit (Qiagen, Germany). The extracted tissue RNA was used to synthesize cDNA using a cDNA synthesis kit (Takara, Japan). Real-time PCR quantitative analysis was performed using an ABI 7500 real-time thermocycler (Biosystems, Foster City, CA, USA) using the same method as in Example 4-3 to determine the expression level of Neuropeptide Y (NPY), a stress-related factor in adipose tissue. The primer information used for PCR is shown in Table 7 below.

**Table 7**

| Gene | Sequence information (5' → 3') | | Sequence No. |
|---|---|---|---|
| NPY | Forward | agagctgctcgagccaccaatgta | 32 |
| | Reverse | caccaatctgatggcccttcttcac | 33 |

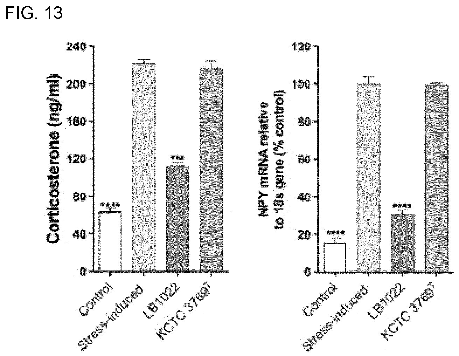

As shown in FIG. 13, compared to the serum corticosterone level of the stress-induced mice (221.61 $\pm$ 4.27 ng/ml), the serum corticosterone level of the mice administrated with *Lactococcus lactis subsp. lactis* KCTC 3769$^T$ strain was 216.89 $\pm$ 7.13 ng/ml. In contrast, mice treated with the novel strain, *Lactococcus lactis subsp. lactis* LB1022 had the serum corticosterone level of 112.09 $\pm$ 4.08 ng/ml. The serum corticosterone level of normal mice was 63.82 $\pm$ 3.47 ng/ml.

Furthermore, compared to the expression level of NPY (100 $\pm$ 3.92%), a stress-related factor in adipose tissue, in mice with induced stress related diabetes, the expression level of NPY in mice administrated with *Lactococcus lactis subsp. lactis* KCTC 3769$^T$ strain was 99.34 $\pm$ 1.55%. In contrast, mice administrated with the novel strain, *Lactococcus lactis subsp. lactis* LB1022 had the NPY expression level of 31.22 $\pm$ 1.54%. The NPY expression level in normal mice was 15.31 $\pm$ 2.92%.

The above results demonstrate that the novel strain, *Lactococcus lactis subsp. lactis* LB1022 has superior prevention and amelioration effects on stress-related diseases compared to the existing type strain, *Lactococcus lactis subsp. lactis* KCTC 3769$^T$.

While the foregoing has described certain aspects of the invention in detail, it will be apparent to one of ordinary skill in the art that these specific descriptions are merely preferred embodiments and are not intended to limit the scope of the invention. That is, the substantive scope of the invention is defined by the appended claims and their equivalents.

The numerical range includes the numbers defined in the range above. All maximum numerical limits given throughout this specification include all lower numerical limits as if the lower numerical limits were clearly written. All minimum numerical limits given throughout this specification include all higher numerical limits as if the higher numerical limits were clearly written. All numerical limits given throughout this specification shall include all better numerical ranges within a wider numerical range, as if the narrower numerical limit were clearly written.

**[Accession number]**

Name of deposit institution: Korea Research Institute of Bioscience and Biotechnology

accession number : KCTC15324BP

deposit date : 2023.02.14

BUDAPEST TREATY ON THE INTERNATIONAL RECOGNITION OF THE DEPOSIT
OF MICROORGANISMS FOR THE PURPOSE OF PATENT PROCEDURE

INTERNATIONAL FORM

# RECEIPT IN THE CASE OF AN ORIGINAL DEPOSIT

issued pursuant to Rule 7.1

TO: KIM, Wonyong

Chung-Ang University

84 Heukseok-ro, Dongjak-gu, Seoul

Republic of Korea

| I. IDENTIFICATION OF THE MICROORGANISM | |
|---|---|
| Identification reference given by the DEPOSITOR: <br><br> *Lactococcus lactis* subsp. *lactis* LB 1022 | Accession number given by the INTERNATIONAL DEPOSITARY AUTHORITY: <br><br> KCTC 15324BP |

| II. SCIENTIFIC DESCRIPTION AND OR PROPOSED TAXONOMIC DESIGNATION |
|---|
| The microorganism identified under I above was accompanied by: <br><br> [ ] a scientific description <br><br> [ ] a proposed taxonomic designation <br><br> (Mark with a cross where applicable) |

| III. RECEIPT AND ACCEPTANCE |
|---|
| This International Depositary Authority accepts the microorganism identified under I above, which was received by it on February 14, 2023. |

| IV. RECEIPT OF REQUEST FOR CONVERSION |
|---|
| The microorganism identified under I above was received by this International Depositary Authority on and a request to convert the original deposit to a deposit under the Budapest Treaty was received by it on |

| V. INTERNATIONAL DEPOSITARY AUTHORITY | |
|---|---|
| Name: Korean Collection for Type Cultures <br><br> Address: Korea Research Institute of Bioscience and Biotechnology (KRIBB) 181, Ipsin-gil, Jeongeup-si, Jeollabuk-do 56212 Republic of Korea | Signature(s) of person(s) having the power to represent the International Depositary Authority or of authorized official(s): <br><br> *Song-Gun Kim* <br><br> KIM, Song-Gun, Director <br> Date: February 14, 2023 |

Form IP/4 (KCTC Form 17)                                                   sole page

**Claims**

1. *Lactococcus lactis subsp. lactis* LB1022 strain deposited under accession number KCTC 15324BP.

2. The strain according to claim 1, wherein the strain has acid tolerance and bile tolerance.

3. The strain according to claim 1, wherein the strain has an ability to adhere to intestinal epithelial cells.

4. The strain according to claim 1, wherein the strain inhibits the production of at least one inflammatory factor selected from the group consisting of nitric oxide (NO), tumor necrosis factor-$\alpha$ (TNF-$\alpha$), interferon-$\gamma$ (IFN-$\gamma$), and interleukin-1$\beta$ (IL-1$\beta$).

5. The strain according to claim 1, wherein the strain inhibits gene expression of p65 or p50 in the Nuclear Factor Kappa B(NF-kB) pathway.

6. A pharmaceutical composition for the prevention or treatment of inflammatory diseases, comprising as an active ingredient, live bacteria, dead bacteria, or a culture medium thereof of the strain of any one of claims 1 to 5.

7. A health functional food composition for the prevention or amelioration of inflammatory diseases, comprising as an active ingredient, live bacteria, dead bacteria, or a culture medium thereof the strain of any one of claims 1 to 5.

8. A health functional food composition for the prevention or amelioration of hangovers, comprising as an active ingredient, live bacteria, dead bacteria, or a culture medium thereof of the strain of any one of claims 1 to 5.

9. A health functional food composition for the prevention or amelioration of liver function, comprising as an active ingredient, live bacteria, dead bacteria or a culture medium thereof the strain of any one of claims 1 to 5.

10. A pharmaceutical composition for the prevention or treatment of allergic diseases, comprising as an active ingredient, live bacteria, dead bacteria or a culture medium thereof the strain of any one of claims 1 to 5.

11. The pharmaceutical composition for the prevention or treatment of allergic diseases according to claim 10, wherein the allergic diseases are one or more diseases selected from the group consisting of allergic dermatitis, atopic dermatitis, asthma, and allergic rhinitis.

12. A health functional food composition for the prevention or amelioration of allergic diseases, comprising as an active ingredient, live bacteria, dead bacteria or a culture medium thereof the strain of any one of claims 1 to 5.

13. The health functional food composition for the prevention or improvement of allergic diseases according to claim 12, wherein the allergic diseases are one or more diseases selected from the group consisting of allergic dermatitis, atopic dermatitis, asthma, and allergic rhinitis.

14. A pharmaceutical composition for the prevention or treatment of skin damage, comprising as an active ingredient, live bacteria, dead bacteria or a culture medium thereof the strain of any one of claims 1 to 5.

15. A cosmetic composition for preventing or improving skin damage, comprising as an active ingredient, live bacteria, dead bacteria or a culture medium thereof the strain of any one of claims 1 to 5.

16. A pharmaceutical composition for the prevention or treatment of obesity, comprising as an active ingredient, live bacteria, dead bacteria or a culture medium thereof of the strain of any one of claims 1 to 5.

17. A health functional food composition for the prevention or amelioration of obesity, comprising as an active ingredient, live bacteria, dead bacteria or a culture medium thereof of the strain of any one of claims 1 to 5.

18. A pharmaceutical composition for the prevention or treatment of diabetes, comprising as an active ingredient, live bacteria, dead bacteria or a culture medium thereof of the strain of any one of claims 1 to 5.

19. A health functional food composition for the prevention or amelioration of diabetes, comprising as an active ingredient, live bacteria, dead bacteria or a culture medium thereof of the strain of any one of claims 1 to 5.

20. A pharmaceutical composition for the prevention or treatment of muscle loss, comprising as an active ingredient, live bacteria, dead bacteria or a culture medium thereof of the strain of any one of claims 1 to 5.

21. A health functional food composition for the prevention or amelioration of muscle loss, comprising as an active ingredient, live bacteria, dead bacteria or a culture medium thereof of the strain of any one of claims 1 to 5.

22. A health functional food composition for gastrointestinal protection, comprising as an active ingredient, live bacteria, dead bacteria or a culture medium thereof of the strain of any one of claims 1 to 5.

23. A pharmaceutical composition for the prevention or treatment of gout, comprising as an active ingredient, live bacteria, dead bacteria or a culture medium thereof of the strain of any one of claims 1 to 5.

24. A health functional food composition for the prevention or amelioration of gout, comprising as an active ingredient, live bacteria, dead bacteria or a culture medium thereof of the strain of any one of claims 1 to 5.

25. A pharmaceutical composition for the prevention or treatment of stress-related diseases, comprising as an active ingredient, live bacteria, dead bacteria or a culture medium thereof of the strain of any one of claims 1 to 5.

26. The pharmaceutical composition for the prevention or treatment of stress-related diseases according to claim 25, wherein the related diseases are one or more diseases selected from the group consisting of stress-related gastritis, stress-related diabetes, stress-related headache, stress-related tension, stress-related anxiety, and stress-related depression.

27. The pharmaceutical composition for the prevention or treatment of stress-related diseases according to claim 25, wherein the pharmaceutical composition has a modulating effect to alleviate or reduce stress.

FIG. 1

FIG. 2

FIG. 3a

**a**

FIG. 3b

**b**

FIG. 3c

FIG. 4

FIG. 5a

FIG. 5b

FIG. 6a

a

FIG. 6b

b

FIG. 7

FIG. 8

Control     High-fat-induced     LB1022     KCTC 3769ᵀ

FIG. 9a

FIG. 9b

FIG. 10

FIG. 11

FIG. 12

FIG. 13

**EP 4 667 561 A1**

## INTERNATIONAL SEARCH REPORT

| International application No. |
|---|
| **PCT/KR2024/095338** |

| A. | CLASSIFICATION OF SUBJECT MATTER |
|---|---|

**C12N 1/20**(2006.01)i; **A61K 35/744**(2015.01)i; **A61P 37/08**(2006.01)i; **A61P 3/10**(2006.01)i; **A23L 33/135**(2016.01)i; **A61K 8/99**(2006.01)i; **A61Q 19/00**(2006.01)i; C12R 1/46(2006.01)n

According to International Patent Classification (IPC) or to both national classification and IPC

| B. | FIELDS SEARCHED |
|---|---|

Minimum documentation searched (classification system followed by classification symbols)

C12N 1/20(2006.01); A23L 33/135(2016.01); A23L 7/104(2016.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 락토코커스 락티스 아종 락티스(Lactococcus lactis subsp. lactis), 내산성(acid resistance), 내담즙성(bile resistance), 장 부착능(intestinal adhesion ability), 항염(anti-inflammatory)

| C. | DOCUMENTS CONSIDERED TO BE RELEVANT |
|---|---|

| Category* | Citation of document, with indication, where appropriate, of the relevant passages | Relevant to claim No. |
|---|---|---|
| X | KAWAHARA, Miho et al. Anti-inflammatory properties of fermented soy milk with Lactococcus lactis subsp. lactis S-SU2 in murine macrophage RAW264.7 cells and DSS-induced IBD model mice. Internatio nal Immunopharmacology. 14 April 2015 (Online), vol. 26, pp. 295-303.<br>See abstract; page 297, left column; page 298, right column-page 301, left column and page 302, left column; and figures 1-3 and 5. | 1-7 |
| X | KUDA, Takashi et al. In vitro evaluation of the fermentative, antioxidant, and anti-inflammation properties of Lactococcus lactis subsp. lactis BF3 and Leuconostoc mesenteroides subsp. mesenteroides BF7 isolated from Oncorhynchus keta intestines in Rausu, Japan. Journal of Functional Foods. 2014, vol. 11, pp. 269–277.<br>See abstract; pages 272 and 276; and figures 1-2 and 6. | 1-2,4-7 |
| A | KR 10-2016-0126605 A (MYONGJI UNIVERSITY INDUSTRY AND ACADEMIA COOPERATION FOUNDATION) 02 November 2016 (2016-11-02)<br>See entire document. | 1-7 |

| ✓ | Further documents are listed in the continuation of Box C. | ✓ | See patent family annex. |
|---|---|---|---|

| * | Special categories of cited documents: |
|---|---|
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **20 June 2024** | **21 June 2024** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

## INTERNATIONAL SEARCH REPORT

| International application No. |
| --- |
| **PCT/KR2024/095338** |

**C.    DOCUMENTS CONSIDERED TO BE RELEVANT**

| Category* | Citation of document, with indication, where appropriate, of the relevant passages | Relevant to claim No. |
| --- | --- | --- |
| PX | BAEK, Jihye et al. Potential Anti-Allergy and Immunomodulatory Properties of Lactococcus lactis LB 1022 Observed In Vitro and in an Atopic Dermatitis Mouse Model. J. Microbiol. Biotechnol. 08 March 2023 (Online), vol. 33, no. 6, pp. 823–830.<br>    See abstract; pages 823, 825, 827 and 829; and figures 1-4. | 1-7 |
| PX | KIM, Jong-Hwa et al. Probiotic cheese improves alcohol metabolism and alleviates alcohol-induced liver injury via the SIRT1/AMPK signaling pathway. Journal of Functional Foods. 20 August 2023 (Online). vol. 108, 105736, pp. 1-8.<br>    See abstract; pages 2 and 5-7; and figure 6. | 1,5-7 |

Form PCT/ISA/210 (second sheet) (July 2022)

**INTERNATIONAL SEARCH REPORT**

International application No.

**PCT/KR2024/095338**

| Box No. I | Nucleotide and/or amino acid sequence(s) (Continuation of item 1.c of the first sheet) |
|---|---|

1. With regard to any nucleotide and/or amino acid sequence disclosed in the international application, the international search was carried out on the basis of a sequence listing:

a. ☑ forming part of the international application as filed.

b. ☐ furnished subsequent to the international filing date for the purposes of international search (Rule 13*ter*.1(a)),

    ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

## INTERNATIONAL SEARCH REPORT

| International application No. |
| --- |
| **PCT/KR2024/095338** |

**Box No. III    Observations where unity of invention is lacking (Continuation of item 3 of first sheet)**

This International Searching Authority found multiple inventions in this international application, as follows:

The invention of group 1 (claims 1-7) pertains to: a Lactococcus lactis subsp. Lactis 1022 strain deposited under accession number KCTC 15324BP (claims 1-5); a pharmaceutical composition for preventing or treating an inflammatory disease, comprising a strain as an active ingredient (claim 6); and a health functional food composition for preventing or treating an inflammatory disease (claim 7),
The invention of group 2 (claim 8) pertains to a health functional food composition for preventing or alleviating a hangover, comprising a strain as an active ingredient,
The invention of group 3 (claim 9) pertains to a health functional food composition for preventing or alleviating a liver function, comprising a strain as an active ingredient,
The invention of group 4 (claims 10-13) pertains to: a pharmaceutical composition for preventing or treating an allergic disease, comprising a strain as an active ingredient (claims 10-11); and a health functional food composition for preventing or alleviating allergic diseases (claims 12-13),
The invention of group 5 (claims 14-15) pertains to: a pharmaceutical composition for preventing or treating skin damage, comprising a strain as an active ingredient (claim 14); and a cosmetic composition for preventing or alleviating skin damage (claim 15),
The invention of group 6 (claims 16-17) pertains to: a pharmaceutical composition for preventing or treating obesity, comprising a strain as an active ingredient (claim 16); and a health functional food composition for preventing or alleviating obesity (claim 17),
The invention of group 7 (claims 18-19) pertains to: a pharmaceutical composition for preventing or treating diabetes, comprising a strain as an active ingredient (claim 18); and a health functional food composition for preventing or alleviating diabetes (claim 19),
The invention of group 8 (claims 20-21) pertains to: a pharmaceutical composition for preventing or treating muscle loss, comprising a strain as an active ingredient (claim 20); and a health functional food composition for preventing or alleviating muscle loss (claim 21),
The invention of group 9 (claim 22) pertains to a health functional food composition for protecting stomach, comprising a strain as an active ingredient,
The invention of group 10 (claims 23-24) pertains to: a pharmaceutical composition for preventing or treating gout, comprising a strain as an active ingredient (claim 23); and a health functional food composition for preventing or alleviating gout (claim 24),
The invention of group 11 (claims 25-27) pertains to a pharmaceutical composition for preventing or treating a stress disease, comprising a strain as an active ingredient.

The technical feature shared by the inventions of groups 1-11 pertains to a pharmacological use of Lactococcus lactis subsp. Lactis, but the feature corresponds to a technology found in the cited document (International Immunopharmacology, 14 April 2015 (Online), vol. 26, pp. 295-303).

Therefore, the inventions of groups 1-11 have no common special technical feature which makes a contribution over the prior art under PCT Rule 13.2, and thus lack unity of invention.

Form PCT/ISA/210 (continuation of first sheet) (July 2022)

## INTERNATIONAL SEARCH REPORT

| International application No. |
| --- |
| **PCT/KR2024/095338** |

**Box No. III        Observations where unity of invention is lacking (Continuation of item 3 of first sheet)**

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☑ No required additional search fees were timely paid by the applicant. Consequently, this international search report is restricted to the invention first mentioned in the claims; it is covered by claims Nos.: **claims 1-7**

**Remark on Protest**        ☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet) (July 2022)

## INTERNATIONAL SEARCH REPORT
### Information on patent family members

International application No.

**PCT/KR2024/095338**

| Patent document cited in search report | | Publication date (day/month/year) | Patent family member(s) | | Publication date (day/month/year) |
|---|---|---|---|---|---|
| KR 10-2016-0126605 | A | 02 November 2016 | KR 10-1689746 | B1 | 02 January 2017 |

Form PCT/ISA/210 (patent family annex) (July 2022)